# FASCICULE DE BREVET EUROPEEN

(11) **EP 3 735 161 B1**
(45) Date de publication et mention de la délivrance du brevet: **16.02.2022**
(21) Numéro de dépôt: 18842814.8
(22) Date de dépôt: 27.12.2018
(51) Int. Cl.: A47K 5/12, A61L 9/03, A61H 37/00

(54) **DISPOSITIF DE DISTRIBUTION EXPÉRIENTIEL D'UN PRODUIT COSMÉTIQUE**
ERFAHRUNGSVORRICHTUNG ZUR AUSGABE EINES KOSMETIKPRODUKTS
EXPERIENTIAL DEVICE FOR DISPENSING A COSMETIC PRODUCT

(30) Priorité: 02.01.2018 FR 1850014
(43) Date de publication de la demande: 11.11.2020
(73) Titulaire: Fauconnier, Sébastien, 36230 Neuvy Saint Sepulchre (FR)
(72) Inventeur: Fauconnier, Sébastien, 36230 Neuvy Saint Sepulchre (FR)
(74) Mandataire: Lequien, Philippe
(86) Numéro de dépôt international: PCT/FR2018/053553
(87) Numéro de publication internationale: WO 2019/135045

(56) Documents cités:
- EP-A1- 0 940 110
- WO-A1-2014/163930
- WO-A1-2017/062387
- DE-A1-102005 028 509
- US-A1- 2014 261 710

## Description

Le domaine de l'invention est celui de la conception et de la fabrication de dispositifs de distribution de produit cosmétique. Plus précisément, l'invention concerne un mode de déclenchement de la distribution d'un produit cosmétique.

Dans le domaine de l'invention, on connaît les distributeurs de produits cosmétiques liquides ou semi-liquides. Par exemple, on connaît les distributeurs de savon qui sont rechargés périodiquement avec une réserve de produit prêt à être distribué.

Les distributeurs peuvent être automatisés pour permettre la distribution d'une dose de produit cosmétique lorsqu'une personne positionne sa main sous une buse de distribution.

De tels distributeurs comprennent ainsi :
- un mécanisme de distribution incluant une buse de distribution par laquelle le produit cosmétique est distribué, et
- un capteur de présence pour détecter une main et commander le mécanisme de distribution pour distribuer le produit cosmétique.

De tels distributeurs simplifient la distribution de doses de produit grâce à leur déclenchement sans contact.

On connaît également des appareils de formulation et de distribution d'une préparation cosmétique, destinés à être installés directement chez les particuliers, notamment pour des applications particulières comme le massage d'un bébé par exemple. De tels appareils, notamment décrits dans les documents de brevets EP 1 671 568, US 2011/303695 et WO 2006/134314 sont également à déclenchement sans contact.

Le document de brevet US 2014/261710 divulgue un dispositif de distribution de produit cosmétique comprenant un mécanisme de distribution d'une dose de produit cosmétique par une buse de distribution, des capteurs de présence aptes à détecter un mouvement d'une main, une zone de surface externe et un capteur de présence étant configuré pour détecter: un premier type de présence d'une main d'une durée comprise entre une première durée et une deuxième durée prédéterminées, et un second type de présence d'une main d'une troisième durée inférieure à la première durée prédéterminée, le dispositif intégrant en outre des moyens de gestion électronique reliés au capteur de présence, au mécanisme de distribution et aux moyens de chauffage paramétrés pour transmettre une consigne de distribution au mécanisme de distribution quand le deuxième type de présence est détecté.

Toutefois, l'application de massage d'un bébé requiert des conditions d'exécution particulières comme la nécessité pour l'utilisateur, dans ce cas le masseur ou la masseuse, d'avoir des mains à une température proche de celle du corps du bébé, surtout pour lui éviter la sensation de froid.

Pour cela il est nécessaire que l'utilisateur puisse se chauffer les mains, sans pour autant être éloigné du bébé, ce qui permet de garder une interaction constante avec ce dernier et renforce sa sécurité, notamment en limitant le risque de chute du bébé hors de la table.

Or, actuellement, il n'existe pas de solution satisfaisante répondant à cette attente.

Il existe ainsi un besoin qui vise à améliorer les circonstances d'application d'une dose de produit cosmétique obtenue par l'intermédiaire d'un distributeur.

Ce besoin est notamment marqué pour les bébés auxquels un produit cosmétique doit être appliqué sous forme de massage. En effet, l'application d'un produit peut être perturbante et même désagréable pour un enfant, notamment si les mains du masseur ou de la masseuse ont une température inférieure à celle du bébé. Il est ainsi nécessaire d'éviter de perturber l'enfant et/ou de pouvoir l'apaiser pour que le massage soit une expérience agréable.

L'invention a notamment pour objectif de répondre à ce besoin de l'art antérieur.

Plus précisément, l'invention a pour objectif de proposer un dispositif de distribution de doses d'un produit cosmétique qui permette des conditions favorables pour l'application du produit cosmétique.

L'invention a en outre pour objectif de proposer un tel dispositif qui soit simple à utiliser.

L'invention a par ailleurs pour objectif de proposer un tel dispositif qui soit simple de conception.

Ces objectifs, ainsi que d'autres qui apparaîtront par la suite, sont atteints grâce à l'invention qui a pour objet un dispositif de distribution de produit cosmétique comprenant :
- un mécanisme de distribution d'une dose de produit cosmétique par une buse de distribution ;
- au moins un capteur de présence, apte à détecter un mouvement d'une main ;
- une zone de surface externe destinée à former un chauffe-main, et des moyens de chauffage de la zone de surface externe, le capteur de présence étant configuré pour détecter au moins :
   - un premier type de présence d'une main, d'une durée comprise entre une première durée t₁ et une deuxième durée t₂ prédéterminées, et
   - un second type de présence d'une main, d'une troisième durée t3 inférieure à la première durée t₁ prédéterminée, et le dispositif intégrant en outre des moyens de gestion électronique reliés au capteur de présence, au mécanisme de distribution et aux moyens de chauffage, paramétrés pour transmettre :
      - une consigne de distribution au mécanisme de distribution, quand le premier type de présence est détecté, ou
      - une consigne de préchauffage aux moyens de chauffage, quand le second type de présence est détecté.

Un tel dispositif offre une sécurité particulière au bébé puisque le masseur ou la masseuse n'utilise qu'un unique appareil et peut garder une main au contact du bébé pour le maintenir et éviter sa chute de la table de massage.

En effet, deux types de présences différents peuvent être détectés par un seul capteur ce qui permet d'habituer l'utilisateur à une unique localisation de détection. L'utilisateur n'aura alors qu'à mémoriser différents mouvements pour que le capteur détecte le mouvement et que le distributeur agisse selon sa volonté, soit pour :
- déclencher la distribution,
- lancer le préchauffage, l'utilisateur ayant un les mains libres pendant le temps de préchauffage.

Selon un mode préféré de réalisation :
- la première durée est comprise entre 2 et 3 secondes, et la deuxième durée est comprise entre 5 et 6 secondes, et
- le second type de présence est compris entre 0,1 s et 1,5 seconde.

Avantageusement, le second type de présence détecté est une succession de détections comprises dans un intervalle de temps compris entre 1 et 2 secondes.

Ainsi, il est possible de différencier clairement la demande de chauffage de la demande de distribution du produit cosmétique. La distribution du produit cosmétique peut alors se faire par une présence prolongée de la main de l'utilisateur devant le capteur tandis que le préchauffage peut se faire par la détection de mouvements successifs devant le capteur.

Selon une forme de réalisation avantageuse, le second type de présence est détecté lorsque le capteur de présence détecte au moins deux présences successives.

Ainsi, on évite le déclenchement du chauffe-main lorsque le capteur détecte une présence accidentelle d'une main. Les deux passages successifs relèvent ainsi d'une action volontaire de déclanchement du chauffe-main.

Selon un mode de réalisation particulier, le dispositif intègre en outre des moyens de diffusion aérienne d'une composition d'aromathérapie, et comprend des moyens d'accueil de capsules jetables reliés aux moyens de diffusion aérienne et au mécanisme de distribution, les moyens d'accueil recevant des capsules jetables de préparation cosmétique prête à être distribuée et/ou des capsules jetables de composition d'aromathérapie pour diffusion aérienne.

Selon un aspect avantageux, le dispositif comprend des moyens électroniques communs de déclenchement du mécanisme de distribution et/ou des moyens de diffusion.

Avantageusement, le dispositif comprend :
- des moyens de lecture de codes d'identification susceptibles d'être portés par des capsules insérées dans les moyens d'accueil ;
- des moyens électroniques de gestion de la distribution de produit cosmétique et/ou de la diffusion aérienne d'une composition d'aromathérapie, les moyens électroniques de gestion étant couplés aux moyens de lecture de codes d'identification.

Un tel dispositif de distribution selon l'invention permet d'optimiser les conditions de distribution d'une dose de produit cosmétique, ainsi que les conditions d'application de la dose de produit cosmétique.

En effet, grâce au système de capsules jetables il est aisé de préparer le dispositif pour qu'il soit prêt à distribuer une dose.

Aussi, grâce aux moyens électroniques communs de déclenchement du mécanisme de distribution et des moyens de diffusion, l'utilisateur peut recevoir une dose de produit cosmétique et procéder à un soin (du visage ou du corps) en appliquant la dose de produit cosmétique distribuée, ceci concomitamment avec la diffusion aérienne d'une préparation d'aromathérapie.

Ces moyens électroniques peuvent notamment synchroniser la diffusion aérienne de la composition d'aromathérapie à la distribution de la dose de la préparation cosmétique prête à être distribuée, par exemple en suivant un programme prédéfini ou spécifique à ladite préparation cosmétique sélectionnée par l'utilisateur.

Par exemple, le produit cosmétique peut correspondre aux soins suivant :
- soin de change bébé ;
- soin d'hydratation ;
- soin de relaxation...

La composition d'aromathérapie peut quant à elle correspondre à :
- soin de bronchiolite ;
- soin de relaxation...

De plus, l'hygiène du dispositif est assurée par l'utilisation des capsules puisque le produit cosmétique est encapsulé en petites quantités ce qui permet de garantir son efficacité et de conserver les bienfaits qu'il peut offrir.

Selon un mode de réalisation préférentiel, le dispositif présente un bec de distribution du produit cosmétique prenant la forme d'un col de cygne présentant une extrémité s'étendant vers le bas, la buse de distribution étant située à l'extrémité du col de cygne, et en ce que le capteur de présence est du type sans contact et forme un espace de détection situé dans un creux formé par le col de cygne, le creux étant situé au-dessus d'un plan horizontal dans lequel s'inscrit la buse de distribution.

Grâce à cette forme en col de cygne du dispositif, la distribution d'une dose de produit cosmétique est améliorée.

En effet, cette conception force l'utilisateur à positionner sa main en dessous de l'extrémité du col de cygne tout en creusant la main de manière à présenter la paume prête à accueillir le produit, avec les doigts qui viennent dans le creux du col de cygne, dans la zone de détection du capteur de présence. De cette manière, la main assume une forme particulièrement adéquate pour recueillir la dose de produit cosmétique.

Dans le cas où le produit cosmétique prêt à être distribué est particulièrement fluide (huile, lait, ...), ce mode de réalisation du distributeur (bec de distribution en forme de col de cygne, zone de détection située dans le creux formé par le col de cygne, ...) permet de s'assurer que la dose va être correctement distribuée et recueillie dans le creux de la main de l'utilisateur.

Par ailleurs, un tel design permet de cacher le capteur pour améliorer l'esthétique du dispositif et de matérialiser, tout en la conservant invisible pour les utilisateurs, la zone de détection qui est facilement atteignable :
D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une représentation schématique du dispositif de distribution selon l'invention, selon une vue en perspective et avec son capot ouvert ;
- la figure 2 est une représentation schématique du dispositif selon l'invention avec son capot fermé ;
- les figures 3 et 4 sont des vues latérales du dispositif de distribution ;
- la figure 5 est une représentation schématique du dispositif de distribution selon l'invention, selon une vue de face.

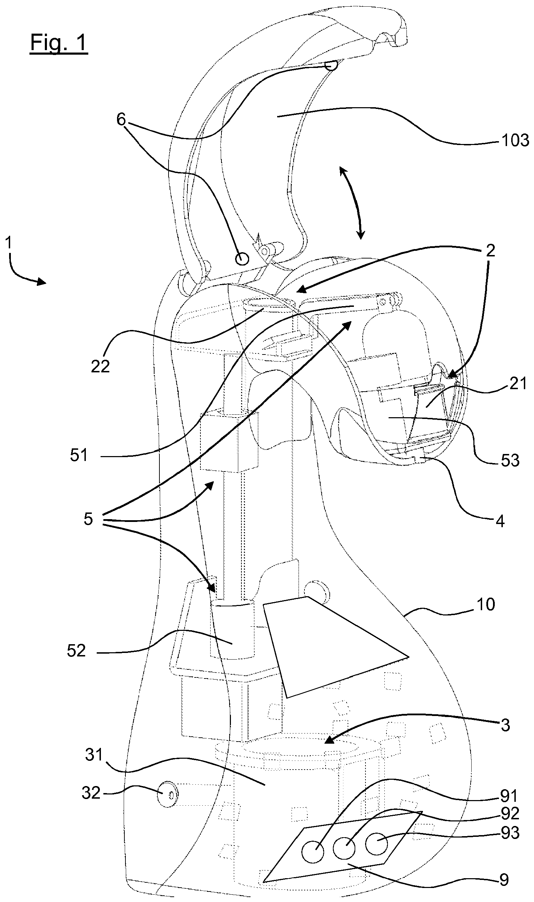

En référence aux figures 1 à 5, le dispositif 1 de distribution selon l'invention comporte une coque 10 présentant une base en partie basse et prenant, dans sa partie haute, la forme d'un col de cygne.

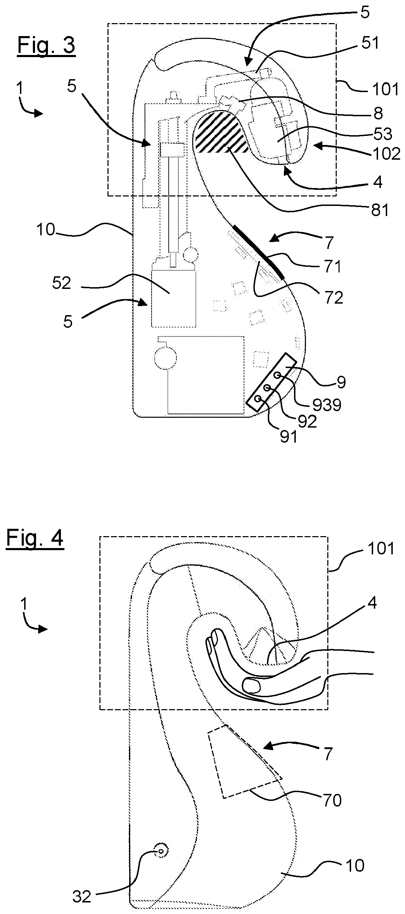

Plus précisément et tel que cela apparaît sur les figures 3 et 4, le dispositif selon l'invention présente dans sa partie haute un bec de distribution 101 de produit cosmétique qui prend la forme d'un col de cygne qui présente une extrémité 102 s'étendant vers le bas.

En référence à la figure 1, le dispositif de distribution selon l'invention comprend :
- des moyens d'accueil 2 de capsules jetables de préparation cosmétique prête à être distribuée et/ou de composition d'aromathérapie pour diffusion aérienne ;
- des moyens de diffusion 3 aérienne d'une composition d'aromathérapie située dans l'une des capsules ;
- une buse de distribution 4 ;
- un mécanisme de distribution 5 d'une dose de produit cosmétique par l'intermédiaire de la buse de distribution 4, à partir de l'une des capsules.

La coque 10 du dispositif de distribution comporte un capot 103 articulé. Ce capot 103 est représenté dans une position ouverte sur la figure 1. Dans la position d'ouverture du capot, les moyens d'accueil 2 de capsules jetables sont accessibles.

En référence à la figure 1, les moyens d'accueil 2 comportent un premier logement 21 (situé à proximité de l'extrémité du col de cygne) pour des capsules de produit cosmétique, et un deuxième logement 22 pour des capsules de composition d'aromathérapie.

Selon le présent mode de réalisation, les capsules destinées à être insérées dans la machine portent des codes d'identification. Tel qu'illustré par la figure 1, le dispositif 1 comprend des moyens de lecture 6 de ces codes d'identification quand les capsules sont insérées dans les moyens d'accueil. Ces moyens de lecture 6, comprenant notamment des capteurs, sont situés sur le capot 103 et viennent en vis-à-vis des capsules insérées dans le premier logement 21 et dans le deuxième logement 22 une fois le capot refermé.

Selon un autre mode de réalisation, les moyens de lecture peuvent comprendre un seul capteur commun à la lecture des codes des deux capsules et situé à l'extérieur du dispositif, par exemple à proximité de la buse de distribution.

Les codes d'identification des capsules peuvent par exemple être des codes-barres, des flash-codes à usage unique, ou encore des codes NFC.

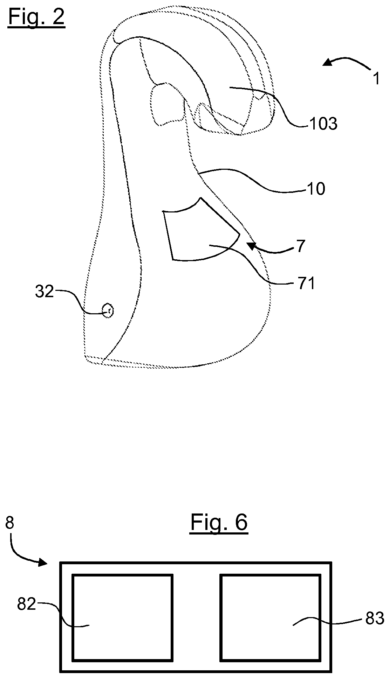

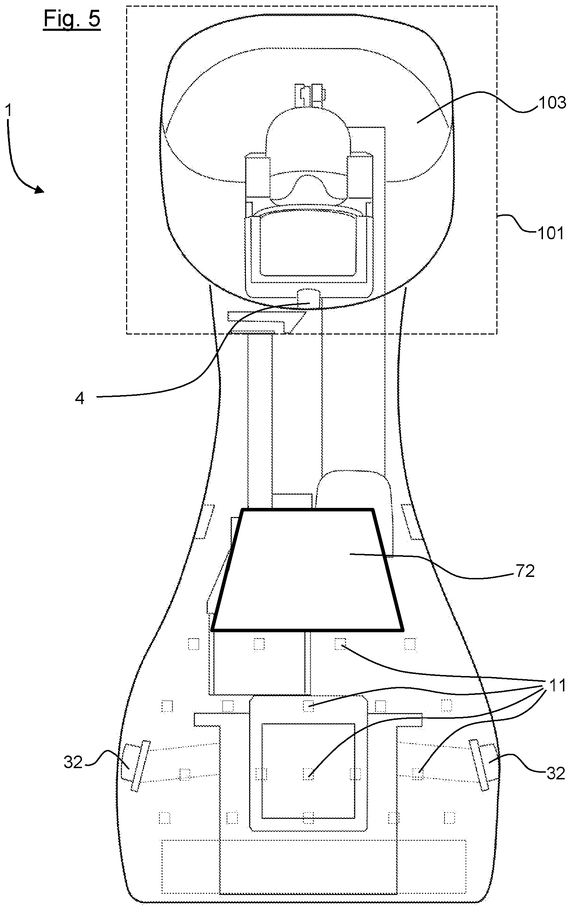

Tel qu'illustré par les figures 2 à 5, le dispositif 1 comprend :
- un chauffe-main 7 formé par une zone de surface externe 71 de la coque 10;
- des moyens de chauffage 72 de la zone de surface externe (représentés sur les figures 3 et 5).

Cette zone de surface externe 71 est située sur la base du dispositif et plus précisément sur la face avant de cette base.

Tel qu'illustré par les figures 3 et 4, le dispositif comprend également un capteur de présence 8 du type « sans contact », positionné à proximité de la buse de distribution 4.

Ce capteur de présence 8 permet de déclencher le mécanisme de distribution 5 lors de la présentation d'une main, ou d'une partie de main devant le capteur de présence 8.

Toujours selon les figures 3 et 4, la buse de distribution 4 est située à l'extrémité 102 du col de cygne, et le capteur de présence 8 « sans contact » est conçu et configuré pour former un espace de détection 81 qui est situé dans un creux formé par la forme du col de cygne, ce creux étant situé au-dessus d'un plan horizontal dans lequel s'inscrit la buse de distribution 4.

Tel qu'illustré sur la figure 4, cette forme particulière du bec de distribution 101 et les emplacements relatifs du capteur de présence 8 et de la buse de distribution 4 forcent un utilisateur qui souhaite avoir une dose de produit cosmétique à recourber sa main pour que ses doigts passent dans l'espace de détection 81.

Grâce à cette configuration, l'utilisateur tend à présenter sa main d'une manière optimisée pour bien recevoir la dose de produit cosmétique et ainsi éviter que le produit cosmétique ne se déverse à côté de la paume de sa main.

En d'autres termes, ce capteur de présence 8 sans contact est positionné en retrait et au-dessus de la buse de distribution 4 par rapport à un sens d'introduction d'une main pour recueillir une dose de produit cosmétique, tout en étant orienté de manière à détecter la présence d'une main sous la buse de distribution 4.

Ce capteur de présence 8 ne déclenche pas une distribution si une personne positionne seulement une partie de sa main juste en dessous de la buse de distribution 4.

Selon le principe de l'invention, le capteur de présence 8 est configuré pour détecter au moins :
- un premier type de présence d'une main, d'une durée comprise entre une première durée t₁ et une deuxième durée t₂ prédéterminées, et
- un second type de présence d'une main, d'une troisième durée t₃ inférieure à la première durée t₁ prédéterminée

De préférence :
- la première durée t₁ est comprise entre 2 et 3 secondes, et la deuxième durée t₂ est comprise entre 5 et 6 secondes, et
- le second type de présence est compris entre 0,1 et 1,5 seconde.

Plus particulièrement, le second type de présence détecté est une succession de détections comprises dans un intervalle de temps lₜ compris entre 1 et 2 secondes.

Pour détecter ce second type de présence, le capteur 8 est configuré pour détecter au moins deux présences successives dont la durée totale, c'est la durée d'une première présence et d'une deuxième présence additionnées, est inférieure à la première durée t₁, mais a minima comprise entre EE et FF secondes. Le détecteur est notamment configuré pour que ces détections successives soient effectuées dans l'intervalle de temps lₜ mentionné précédemment.

Toutefois, bien que deux présences successives soient mentionnées ciavant, il est également possible de configurer le capteur pour détecter plus de deux présences successives.

La succession de mouvements peut être réalisée par un balayage de type aller-retour, c'est-à-dire dans un premier sens puis dans un deuxième sens contraire au premier, ou par deux mouvements dans un même sens.

Selon un mode de réalisation particulier représente à la figure 6, le capteur de présence 8 comprend une première cellule de détection 82 et une deuxième cellule de détection 83 contigües.

Par un capteur 8 disposant de deux cellules 82, 83, le second type de présence est détecté lorsque l'utilisateur réalise un mouvement de main correspondant à un balayage, devant la première cellule et la deuxième cellule qui détectent chacune individuellement une présence.

Le balayage est notamment détecté lorsque le capteur détecte une présence selon une séquence de détection de la première cellule, puis de détection de la deuxième cellule, et inversement.

Selon le présent mode de réalisation illustré par les figures 1 et 3, le mécanisme de distribution 5 d'une dose de produit cosmétique comprend :
- un mécanisme à piston 51 actionné par un moteur 52 ;
- un circuit de chauffage 53 de la dose à distribuer.

Le circuit de chauffage 53 permet d'amener une dose de produit cosmétique à distribuer à température corporelle.

Les moyens de diffusion 3 aérienne d'une composition d'aromathérapie sont du type à ultra-sons.

En référence à la figure 1, lors de l'insertion d'une capsule de composition d'aromathérapie dans le deuxième logement 22 des moyens d'accueil, une aiguille vient perforer la capsule pour amorcer les moyens de diffusion 3. Lors d'un déclenchement des moyens de diffusion 3, des gouttelettes de la composition d'aromathérapie s'écoulent de la capsule de composition d'aromathérapie jusque dans un bloc 31 où elles sont mises en suspension dans l'air, sous la forme d'une fumée, à l'aide d'une membrane à ultra-sons. Des ventilateurs 32 permettent de créer un flux d'air pour augmenter la diffusion de la composition d'aromathérapie mise en suspension dans l'air.

En référence aux figures 1 et 3, le dispositif comprend également une carte électronique 9. Cette carte électronique regroupe :
- des moyens électroniques communs de déclenchement 91 du mécanisme de distribution 5 et/ou des moyens de diffusion 3 ;
- des moyens électroniques de gestion 92 de la distribution de produit cosmétique et/ou de la diffusion aérienne d'une composition d'aromathérapie ;
- des moyens électroniques de communication sans fil 93.

Grâce aux moyens électroniques communs de déclenchement 91, la distribution d'une dose de produit cosmétique et la diffusion aérienne d'une composition d'aromathérapie peut être synchronisée, ou bien réalisée de manière complémentaire pour obtenir de manière particulièrement aisée une expérience sensorielle satisfaisante.

Les moyens électroniques de gestion 92 sont couplés aux moyens de lecture 6 de codes d'identification. Ces moyens électroniques de gestion 92 permettent d'adapter la distribution de produit cosmétique et la diffusion aérienne d'une composition d'aromathérapie aux produits et compositions des capsules insérées dans le dispositif 1.

En effet, les codes d'identification permettent aux moyens de paramétrage d'intégrer, par exemple, la contenance des capsules (6 mL, 12 mL, ...) et le nombre de doses disponibles dans les capsules. Selon les codes d'identification, d'autres paramètres du dispositif selon l'invention peuvent être modifiés tel que la température de chauffage de la dose de produit cosmétique à distribuer.

Une sécurité, apte à verrouiller le dispositif 1, peut être installée pour empêcher l'utilisation de produits cosmétiques et de compositions d'aromathérapie non conformes. Les codes d'identifications servent alors à déverrouiller le dispositif 1.

Les moyens électroniques de communication sans-fil 93 permettent au dispositif de distribution 1 selon l'invention d'interagir avec d'autres dispositifs électroniques.

Par exemple, le dispositif de distribution 1 peut être appairé avec un ordiphone ou une tablette électronique pour contrôler le dispositif de distribution 1 à distance, ou recevoir des informations du dispositif 1.

L'ordiphone ou la tablette électronique peuvent être pourvus d'une application dédiée au dispositif de distribution.

Selon un exemple pratique, l'application peut afficher des combinaisons de compositions d'aromathérapie et de produits cosmétique à utiliser en synergie.

Lors de l'insertion des capsules relatives dans le dispositif de distribution 1, les codes d'identification permettent de s'assurer que les bons produits ont été insérés dans les moyens d'accueil du dispositif, et les moyens électroniques de communication sans fil peuvent alors envoyer une information relative à ces produits. L'application peut alors afficher automatiquement une vidéo de démonstration expliquant la méthode à suivre pour bien appliquer les soins.

L'application peut également gérer les stocks de capsules en répertoriant les capsules utilisées, puis permettre de réaliser une nouvelle commande de capsules en prenant en compte ce qui a été utilisé.

En référence aux figures 2, 4 et 5, le dispositif 1 comporte en outre des haut-parleurs 10 et des LEDs de luminothérapie 11 (illustrées sur la figure 5).

Les LEDs de luminothérapie 11 et les haut-parleurs 10 peuvent par exemple être déclenchés lors de la réalisation d'un programme particulier (un massage par exemple). Les haut-parleurs 10 peuvent alors diffuser automatiquement des sons ou des musiques relaxantes adaptés au type de massage réalisé. De même, suivant le massage, Les LEDs de luminothérapie 11 peuvent alors suivre une séquence précise pour optimiser l'effet relaxant du massage.

L'utilisateur peut utiliser une tablette électronique sur laquelle il a préalablement installé l'application information dédiée au dispositif 1, et qu'il a appairé au dispositif 1.

Sur l'application, l'utilisateur peut par exemple sélectionner un programme de massage qu'il souhaite réaliser.

Dans le cadre de l'application d'un produit cosmétique (un soin) à un bébé par sa mère, l'application peut indiquer à la maman une combinaison adaptée comportant une capsule de composition d'aromathérapie relaxante et une capsule de produit cosmétique prêt à l'emploi.

Suite à l'insertion des capsules dans le dispositif 1, ce dernier se paramètre automatiquement et une vidéo de démonstration peut être affichée sur la tablette électronique pour expliquer la manière optimale de réaliser le soin.

La mère peut alors débuter le soin en se réchauffant les mains à l'aide du chauffe-main, en récupérant la dose de produit cosmétique réchauffée à température corporelle, puis en commençant à réaliser l'application, par exemple un massage.

Lors de la réalisation de ce soin, le dispositif selon l'invention déclenche automatiquement la diffusion aérienne de la composition d'aromathérapie, crée une ambiance lumineuse synchronisée avec le massage par l'intermédiaire des LEDs de luminothérapie 11 et diffuse également des bruits ou une musique relaxante également synchronisée avec le soin.

En fonctionnement, l'utilisateur peut ainsi choisir :
- de commander le préchauffage des moyens de chauffage 72 du chauffe-main 7 en réalisant avec une seule main un mouvement de balayage devant le capteur de présence 8, ou
- de commander le mécanisme de distribution 5 pour obtenir la distribution de produit cosmétique par la buse de distribution 4.

Le dispositif 1 selon l'invention permet ainsi d'obtenir de manière simple une distribution et une application expérientielles d'un produit cosmétique tout en autorisant une utilisation par une seule main pour garantir la sécurité du bébé sur la table de massage en gardant un contact permanent avec lui.

## Revendications

1. Dispositif de distribution (1) de produit cosmétique comprenant :
- un mécanisme de distribution (5) d'une dose de produit cosmétique par une buse de distribution (4) ;
- au moins un capteur de présence (8), apte à détecter un mouvement d'une main ;
- une zone de surface externe (71) destinée à former un chauffe-main (7), et des moyens de chauffage (72) de la zone de surface externe (71), le capteur de présence (8) étant configuré pour détecter au moins :
- un premier type de présence d'une main, d'une durée comprise entre une première durée t₁ et une deuxième durée t₂ prédéterminées, et
- un second type de présence d'une main, d'une troisième durée t₃ inférieure à la première durée t₁ prédéterminée, et le dispositif intégrant en outre des moyens de gestion électronique (92) reliés au capteur de présence (8), au mécanisme de distribution (5) et aux moyens de chauffage (72), paramétrés pour transmettre :
- une consigne de distribution au mécanisme de distribution (5), quand le premier type de présence est détecté, ou
- une consigne de préchauffage aux moyens de chauffage (72), quand le second type de présence est détecté.

2. Dispositif (1) selon la revendication 1, la première durée t₁ étant comprise entre 2 et 3 secondes, et la deuxième durée t₂ étant comprise entre 5 et 6 secondes.

3. Dispositif (1) selon la revendication 1 ou 2, le second type de présence étant compris entre 0,1 et 1,5 seconde.

4. Dispositif (1) selon l'une quelconque des revendications précédentes, second type de présence détecté étant une succession de détections comprises dans un intervalle de temps lₜ compris entre 1 et 2 secondes.

5. Dispositif (1) selon l'une quelconque des revendications précédentes, second type de présence étant détecté lorsque le capteur de présence (8) détecte au moins deux présence présences successives.

6. Dispositif (1) selon les revendications 4 et 5, le second type de présence étant détecté lorsque le capteur de présence (8) détecte une présence selon une séquence de détection de la première cellule (81), puis de détection de la deuxième cellule (82), et inversement.

7. Dispositif (1) selon l'une quelconque des revendications précédentes, le dispositif intégrant en outre des moyens de diffusion aérienne (3) d'une composition d'aromathérapie, et comprenant des moyens d'accueil (2) de capsules jetables reliés aux moyens de diffusion aérienne (3) et au mécanisme de distribution (5), les moyens d'accueil recevant des capsules jetables de préparation cosmétique prête à être distribuée et/ou des capsules jetables de composition d'aromathérapie pour diffusion aérienne.

8. Dispositif (1) selon la revendication précédente, le dispositif comprenant des moyens électroniques communs de déclenchement (91) du mécanisme de distribution (5) et/ou des moyens de diffusion (5).

9. Dispositif (1) selon la revendication précédente, le dispositif comprenant:
- des moyens de lecture (6) de codes d'identification susceptibles d'être portés par des capsules insérées dans les moyens d'accueil (2) ;
- des moyens électroniques de gestion (92) de la distribution de produit cosmétique et/ou de la diffusion aérienne d'une composition d'aromathérapie, les moyens électroniques de gestion (92) étant couplés aux moyens de lecture (6) de codes d'identification.

10. Dispositif (1) selon l'une quelconque des revendications précédentes, le dispositif présentant un bec de distribution (101) du produit cosmétique prenant la forme d'un col de cygne présentant une extrémité (102) s'étendant vers le bas, la buse de distribution (4) étant située à l'extrémité du col de cygne, capteur (8) de présence étant du type sans contact et forme un espace de détection (81) situé dans un creux formé par le col de cygne, le creux étant situé au-dessus d'un plan horizontal dans lequel s'inscrit la buse de distribution (4).

## Patentansprüche

1. Vorrichtung zur Ausgabe (1) eines Kosmetikprodukts, umfassend:
- einen Ausgabemechanismus (5) einer Dosis eines Kosmetikprodukts durch eine Ausgabedüse (4);
- mindestens einen Anwesenheitssensor (8), der imstande ist, eine Bewegung einer Hand zu erkennen;
- einen äußeren Oberflächenbereich (71), der dazu bestimmt ist, einen Handwärmer (7) zu bilden, und Heizmittel (72) für den äußeren Oberflächenbereich (71),
wobei der Anwesenheitssensor (8) konfiguriert ist, um mindestens Folgendes zu erkennen:
- eine erste Anwesenheitsart einer Hand, von einer Dauer, die zwischen einer ersten vorbestimmten Dauer t₁ und einer zweiten vorbestimmten Dauer t₂ liegt, und
- eine zweite Anwesenheitsart einer Hand, von einer dritten Dauer t₃ kürzer als die erste vorbestimmte Dauer t₁, und wobei die Vorrichtung weiter elektronische Verwaltungsmittel (92) integriert, die an den Anwesenheitssensor (8), den Ausgabemechanismus (5) und die Heizmittel (72) angeschlossen sind, parametriert, um Folgendes zu übertragen:
- eine Ausgabevorgabe an den Ausgabemechanismus (5), wenn die erste Anwesenheitsart erkannt wird, oder
- eine Vorheizvorgabe an die Heizmittel (72), wenn die zweite Anwesenheitsart erkannt wird.

2. Vorrichtung (1) nach Anspruch 1, wobei die erste Dauer t₁ zwischen 2 und 3 Sekunden liegt, und die zweite Dauer t₂ zwischen 5 und 6 Sekunden liegt.

3. Vorrichtung (1) nach Anspruch 1 oder 2, wobei die zweite Anwesenheitsart zwischen 0,1 und 1,5 Sekunden liegt.

4. Vorrichtung (1) nach einem der vorstehenden Ansprüche, wobei die zweite erkannte Anwesenheitsart eine Aufeinanderfolge von Erkennungen ist, die in einem Zeitintervall lₜ liegt, das zwischen 1 und 2 Sekunden liegt.

5. Vorrichtung (1) nach einem der vorstehenden Ansprüche, wobei die zweite Anwesenheitsart erkannt wird, wenn der Anwesenheitssensor (8) mindestens zwei aufeinanderfolgende Anwesenheit Anwesenheiten erkennt.

6. Vorrichtung (1) nach den Ansprüchen 4 und 5, wobei die zweite Anwesenheitsart erkannt wird, wenn der Anwesenheitssensor (8) eine Anwesenheit gemäß einer Folge einer Erkennung der ersten Zelle (81), danach einer Erkennung der zweiten Zelle (82), und umgekehrt erkennt.

7. Vorrichtung (1) nach einem der vorstehenden Ansprüche, wobei die Vorrichtung weiter Diffusionsmittel über Luft (3) einer Aromatherapie-Zusammensetzung integriert und Aufnahmemittel (2) für Einwegkapseln umfasst, die an die Diffusionsmittel über Luft (3) und an den Ausgabemechanismus (5) angeschlossen sind, wobei die Aufnahmemittel Einwegkapseln einer ausgabebereiten kosmetischen Präparation und/oder Einwegkapseln einer Aromatherapie-Zusammensetzung zur Diffusion über Luft empfangen.

8. Vorrichtung (1) nach dem vorstehenden Anspruch, wobei die Vorrichtung gemeinsame elektronische Auslösemittel (91) des Ausgabemechanismus (5) und/oder der Diffusionsmittel (5) umfasst.

9. Vorrichtung (1) nach dem vorstehenden Anspruch, wobei die Vorrichtung umfasst:
- Mittel zum Lesen (6) von Identifikationscodes, die imstande sind, von den in die Aufnahmemittel (2) eingeführten Kapseln getragen zu werden;
- elektronische Verwaltungsmittel (92) für die Ausgabe eines kosmetischen Produkts und/oder die Diffusion über Luft einer Aromatherapie-Zusammensetzung, wobei die elektronischen Verwaltungsmittel (92) mit den Mitteln zum Lesen (6) von Identifikationscodes gekoppelt sind.

10. Vorrichtung (1) nach einem der vorstehenden Ansprüche, wobei die Vorrichtung eine Ausgabetülle (101) für das kosmetische Produkt aufweist, die die Form eines Schwanenhalses annimmt, der ein Ende (102) aufweist, das sich nach unten erstreckt, wobei sich die Ausgabedüse (4) am Ende des Schwanenhalses befindet, der Anwesenheitssensor (8) von der kontaktlosen Art ist, und einen Erkennungsraum (81) bildet, der sich in einem Hohlraum befindet, der durch den Schwanenhals gebildet wird, wobei sich der Hohlraum oberhalb einer horizontalen Ebene befindet, in der die Ausgabedüse (4) eingeschrieben ist.

## Claims

1. A cosmetic product dispensing device (1) comprising:
- a mechanism (5) for dispensing a dose of a cosmetic product through a dispensing nozzle (4);
- at least one presence sensor (8), adapted to detect a movement of a hand;
- an outer surface area (71) intended to form a hand warmer (7), and means (72) for heating the outer surface area (71),
the presence sensor (8) being configured to detect at least:
- a first type of presence of a hand, with a duration comprised between a predetermined first duration t₁ and a second duration t₂, and
- a second type of presence of a hand, with a third duration t₃ shorter than the first predetermined duration t₁, and the device further integrating electronic management means (92) connected to the presence sensor (8), to the dispensing mechanism (5) and to the heating means (72), parameterised to transmit:
- a dispensing command to the dispensing mechanism (5), when the first presence type is detected, or
- a preheating command to the heating means (72), when the second presence type is detected.

2. The device (1) according to claim 1, the first duration t₁ being comprised between 2 and 3 seconds, and the second duration t₂ being comprised between 5 and 6 seconds.

3. The device (1) according to claim 1 or 2, the second presence type being comprised between 0.1 and 1.5 seconds.

4. The device (1) according to any one of the preceding claims, the second detected presence type being a succession of detections comprised within a time interval Iₜ comprised between 1 and 2 seconds.

5. The device (1) according to any one of the preceding claims, the second presence type being detected when the presence sensor (8) detects at least two successive presences.

6. The device (1) according to claims 4 and 5, the second presence type being detected when the presence sensor (8) detects a presence according to a sequence of detection of the first cell (81), then of detection of the second cell (82), and vice versa.

7. The device (1) according to any one of the preceding claims, the device further integrating means for air diffusion (3) of an aromatherapy composition, and comprising means (2) for receiving disposable capsules connected to the aerial diffusion means (3) and to the dispensing mechanism (5), the reception means receiving disposable capsules of a cosmetic preparation ready to be dispensed and/or disposable capsules of an aromatherapy composition for aerial diffusion.

8. The device (1) according to the preceding claim, the device comprising common electronic means (91) for triggering the dispensing mechanism (5) and/or the diffusion means (5).

9. The device(1) according to the preceding claim, the device comprising:
- means (6) for reading identification codes that might be carried by capsules inserted in the reception means (2) ;
- electronic means (92) for managing the dispense of a cosmetic product and/or of the aerial diffusion of an aromatherapy composition, the electronic management means (92) being coupled to the means (6) for reading identification codes.

10. The device (1) according to any one of the preceding claims, the device having a spout (101) for dispensing the cosmetic product shaped like a swan neck having an end (102) extending downwards, the dispensing nozzle (4) being located at the end of the swan neck, and the presence sensor (8) being of the contactless type and forms a detection space (81) located in a recess formed by the swan neck, the recess being located above a horizontal plane in which the dispensing nozzle (4) fits.
